# EUROPÄISCHE PATENTSCHRIFT

(11) **EP 1 843 803 B1**
(45) Veröffentlichungstag und Bekanntmachung des Hinweises auf die Patenterteilung: **08.01.2014**
(21) Anmeldenummer: 05769676.7
(22) Anmeldetag: 18.07.2005
(51) Int. Cl.: A61L 27/38

(54) **TRANSPLANTATKONSTRUKT MIT TERMINAL DIFFERENZIERTEN GEWEBEZELLEN ZUR REKONSTRUKTION EINES ORGANS**
GRAFT STRUCTURE COMPRISING TERMINALLY DIFFERENTIATED TISSUE CELLS FOR THE RECONSTRUCTION OF AN ORGAN
STRUCTURE DE GREFFE TISSULAIRE POUR RECONSTITUER UN ORGANE HUMAIN OU ANIMAL

(30) Priorität: 30.07.2004 DE 102004037184
(43) Veröffentlichungstag der Anmeldung: 17.10.2007
(73) Patentinhaber: UroTiss GmbH, 01069 Dresden (DE)
(72) Erfinder: RAM-LIEBIG, Gouya, 01309 Dresden (DE); HAKENBERG, Oliver, 01309 Dresden (DE); WIRTH, Manfred, 01326 Dresden (DE)
(74) Vertreter: Carlsohn, Alexander
(86) Internationale Anmeldenummer: PCT/DE2005/001266
(87) Internationale Veröffentlichungsnummer: WO 2006/012837

(56) Entgegenhaltungen:
- WO-A-98/06445
- US-B1- 6 428 802
- SCRIVEN S D ET AL: "RECONSTITUTION OF HUMAN UROTHELIUM FROM MONOLAYER CULTURES" JOURNAL OF UROLOGY, BALTIMORE, MD, US, Bd. 158, Nr. 3, PART 2, September 1997 (1997-09), Seiten 1147-1152, XP009025877 ISSN: 0022-5347
- YOO JAMES J ET AL.: "Bladder augmentation using allogenic bladder submucosa seeded with cells" UROLOGY, Bd. 51, Nr. 2, Februar 1998 (1998-02), Seiten 221-225, XP002362463
- ATALA A: "TISSUE ENGINEERING FOR BLADDER SUBSTITUTION" WORLD JOURNAL OF UROLOGY, SPRINGER INTERNATIONAL, DE, Bd. 18, Nr. 5, Oktober 2000 (2000-10), Seiten 364-370, XP000985540 ISSN: 0724-4983

## Beschreibung

Die Erfindung betrifft ein Gewebetransplantatkonstrukt zur Rekonstruktion eines menschlichen oder tierischen Organs, ein Verfahren zu dessen Herstellung sowie eine Verwendung des Gewebetransplantatkonstruktes. Die Erfindung betrifft insbesondere ein Gewebetransplantatkonstrukt zur Rekonstruktion der Harnblase.

Die Rekonstruktion der Harnblase kann aufgrund angeborener oder erworbener Erkrankungen wie beispielsweise Meningomyelozele, Blasen- und Kloakaldystrophy, Traumata, Malignome, neuropathische Dysfunktionen, Detrusorinstabilität notwendig sein. Die Rekonstruktion, d. h. der Ersatz beschädigter oder erkrankter Bereiche einer Harnblase, erfolgt mittels Gewebetransplantatkonstrukten, die als eine Matrix für das nachwachsende ursprüngliche Gewebe dienen sollen.

Gegenwärtig werden hauptsächlich patienteneigene gastrointestinal-Segmente als Matrix verwendet. Alternativ, aber weniger häufig werden patienteneigene de-epithelialisierte Darmsegmente eingesetzt. Diese Gewebetransplantatkonstrukte weisen jedoch schwerwiegende Nachteile auf. So ist die Benutzung von Gastrointestinal-Segmenten mit Komplikationen wie beispielsweise Elektrolytstörung, Mukusproduktion, Steinbildung oder maligne Entartung assoziiert. Der Grund dafür ist der Dauerkontakt von Gastrointestinalschleimhaut mit Urin. Die Benutzung von de-epithelialisierten Darmsegmenten verursacht Komplikationen wie Schrumpfung des Segmentes.

Während Matrices ohne Zellen für die Rekonstruktion von kleineren Defekten einer Harnblase geeignet sind, ist eine Beschichtung der Matrix bei größeren Defekten vorteilhaft (Yoo JJ, Meng J, Oberpenning F, Atala A. Bladder augmentation using allogenic bladder submucosa seeded with cells. Urology. 1998 Feb;51(2):221-5). Es ist vorgeschlagen worden, eine Matrix, die zur Rekonstruktion einer Harnblase verwendet werden soll, mit Urothelzellen zu beschichten. Auf diese Weise soll die Matrix gegen die zersetzende Wirkung des Urins geschützt werden, da nur Urothelzellen eine geeignete Barriere gegen Urin bilden und die Matrix somit gegen Urin und außerdem Verkalkung und Abstoßung schützen.

Ferner ist bekannt, daß nur terminal differenzierte superfizielle Zellen der Urothelialschicht die entsprechende Funktionalität gewährleisten und insbesondere eine Barriere gegen Urin und seine Komponente bilden. Die Infiltration der toxischen Substanzen des Urins wird nämlich von terminal differenzierten Urothelzellen gewährleistet, die somit für die Aufrechterhaltung des osmotischen Gradienten der Harnblase und der Gewebehomeoestase essentiell sind.

Weniger differenzierte Urothelzellen bieten jedoch nicht die Funktionalität terminal differenzierter Urothelzellen. Ein Gewebetransplantatkonstrukt aus einer Matrix, die mit weniger differenzierten Urothelzellen beschichtet ist, kann somit aufgrund der mangelhaften Funktion dieser Urothelzellen unter anderem zur Infektion, Verkalkung, und Abstoßung der Matrix führen.

Um eine Abstoßung der mit Urothelzellen beschichteten Matrix zu vermeiden, wäre die Verwendung autologer Urothelzellen von Vorteil. Allerdings ist dies mit Schwierigkeiten verbunden: Durch Biopsie aus einer alten und erkrankten Blase gewonnene Urothelzellen weisen nur ein eingeschränktes Regenerationspotential auf (Cheng EY, Kropp BP. Urologic tissue engineering with small-intestinal submucosa: potential clinical applications. World J Urol. 2000 Feb; 18(1):26-30). Überdies verlieren die so erhaltenen Urothelzellen während der Proliferation ihren charakteristischen in vivo Phänotyp (Von der Zellkultur zum Tissue engineering: W.W: Minuth, R. Strehl, K. Schumacher. Pabst science publishers), so daß es schwierig ist, diese Urothelzellen zu normal funktionierenden, hoch differenzierten Zellen zu redifferenzieren.

Lebensfähige autologe Gewebekonstrukte sind aus [1-5] bekannt. Die begrenzte Regenerierungsfähigkeit von Urothelzellen, die aus alten und kranken Harnblasen gewonnen worden, ist aus [6] bekannt. Der Verlust des charakteristischen Phänotyps von Urothelzellen während der Proliferation in vivo wird in [7] beschrieben. Der Einfluß der extrazellulären Matrix wird in [8-16] näher erläutert.

WO 98/06445 A1 offenbart ein Verfahren zur Rekonstruktion von Gewebe durch Besiedlung von Harnblasen-Submocosa mit Zellen.

Scriven et al. J Urol 1997, 158: 1147-52, offenbaren die Kultur von Urothelzellen auf de-epithelisierten Urothelstroma.

Aufgabe der Erfindung ist es, die Nachteile nach dem Stand der Technik zu beseitigen. Es soll insbesondere ein Gewebetransplantatkonstrukt zur Rekonstruktion eines menschlichen oder tierischen Organs, insbesondere einer Harnblase, angegeben werden, das weitestgehend dieselben schützende Funktionscharakteristika wie das gesunde native Urothelialgewebe aufweist und somit die weitere Generierung der Muskelschicht über die Membran nach der Implantation unterstützt. Ferner sollen ein Verfahren zu Herstellung einer solchen Gewebe-transplantatkonstruktes sowie eine Verwendung des Gewebetransplantatkonstruktes angegeben werden.

Diese Aufgabe wird durch die Merkmale der Ansprüche 1, 7 und 12 gelöst. Zweckmäßige Ausgestaltungen der Erfindungen ergeben sich aus den Merkmalen der Unteransprüche.

Nach Maßgabe der Erfindung ist ein Gewebetransplantatkonstrukt zur Rekonstruktion einer menschlichen oder tierischen Harnblase vorgesehen, das eine biologisch kompatible, kollagenhaltige Membran und eine oder mehrere Schichten von organspezifischen Gewebezellen auf der Membran umfaßt, wobei die äußere Schicht der organ-spezifischen Gewebezellen eine Schicht terminal differenzierter organ-spezifischer Gewebezellen ist wobei die organspezifischen Gewebezellen Urothelzellen sind.

Der Begriff "organ-spezifische Gewebezellen" soll hierbei so verstanden werden, daß Gewebezellen desselben oder eines gleichen Organs, das rekonstruiert werden soll, auf die Membran aufgebracht werden. Soll beispielsweise eine Harnblase rekonstruiert werden, so sind hier unter organ-spezifischen Gewebezellen Urothelzellen zu verstehen.

Organe sind funktionelle Einheiten des Körpers. Bevorzugtes Beispiel ist die Harnblase.

In einer bevorzugten Ausführungsform ist ein Gewebetransplantatkonstrukt zur Rekonstruktion der Harnblase vorgesehen, das eine biologisch kompatible, kollagenhaltige Membran und ein oder mehrere Schichten von Urothelzellen auf der Membran umfaßt, wobei die äußere Schicht der Urothelzellen eine Schicht terminal differenzierter Urothelzellen ist.

Ist auf der Membran nur eine Schicht an Gewebezellen ausgebildet, so stellt diese Schicht die äußere Schicht dar, die eine Schicht terminal differenzierter Gewebezellen ist.

Die äußere Schicht ist eine Schicht terminal differenzierter Gewebezellen. Die übrigen Schichten sind Schichten nichtterminal differenzierter Gewebezellen.

Die Begriffe "Membran" und "Matrix" werden hier, soweit nicht anders angegeben, synonym verwendet. Die Membran sollte die Komponenten der extrazellulären Matrix (EZM), insbesondere deren Wachstumsfaktoren, enthalten. Vorzugsweise ist die Membran eine biologische Membran.

Unter einer kollagenhaltigen Membran soll hier eine Membran auf Kollagenbasis verstanden werden.

Unter Rekonstruktion wird der Ersatz von beschädigten oder kranken Bereichen einer menschlichen oder tierischen Harnblase verstanden.

Unter stromaler Induktion wird die durch Stroma induzierte Proliferation der Gewebezellen verstanden. Vorzugsweise ist die stromale Induktion eine fibroblastische Induktion. Die stromale Induktion soll eine Proliferation der Gewebezellen ermöglichen, die ausreicht, um größere Membranflächen (bis zu 40 cm²) zu bedecken.

Die Zahl der Schichten von Gewebezellen auf der Membran sollte vorzugsweise ein bis sieben, stärker bevorzugt zwei bis sieben, am stärksten bevorzugt vier bis sieben betragen, wobei die äußere Schicht eine terminal differenzierte Gewebezell-Schicht ist. Wenn das Konstrukt zur Rekonstruktion eines menschlichen Organs verwendet werden soll, sind die Gewebezellen vorzugsweise autologen humanen Ursprungs. Vorzugsweise ist nur die äußere Schicht eine Schicht von terminal differenzierten Gewebezellen.

Innerhalb von Geweben und Organen wird die Zellkommunikation durch das komplexe Molekulargerüst der extrazellulären Matrix (EZM) ermöglicht. Biochemische und biophysikalische Signale aus der extrazellulären Matrix sind unter anderem fundamental an der Regulierung von zellulären Aktivitäten wie Adhäsion, Formbildung, Migration, Proliferation, Differenzierung und Funktionsbildung beteiligt. Auch in der Harnblase ist die Funktion der extrazellulären Matrix und ihre Interaktion mit den Zellen bezüglich der Entwicklung, Regeneration und Pathogenese sehr bedeutend. Zum Beispiel bewirken altersbedingte Faktoren Änderungen in der Ultrastruktur der extrazellulären Matrix und somit in dem Regenerationspotential des Blasengewebes.

Die biologisch kompatible, kollagenhaltige Membran ist vorzugsweise ein Gewebe, das von einem Säuger stammt, insbesondere ein humanes oder porzines (Schweine-) submukosales oder dermales Gewebe. Die Gewinnung dieses Gewebes ist bekannt.

Die Wahl der geeigneten Membran mit den entsprechenden Komponenten der extrazellulären Matrix und die spezifischen Signalprozesse dieser Komponenten sind somit bezüglich ihrer orientierenden und regulierenden Funktion die erste Voraussetzung für den Erfolg der Zellexpansion und Differenzierung der epithelialen Zellen.

Die Vorbereitung der azellulären Membranen sollte daher eine komplette Entfernung der wichtigen Wachstumsfaktoren und anderer Komponenten der extrazellulären Matrix vermeiden, um ein möglichst natürliches Milieu für die Zellkultur bereitzustellen und bi-direktionale und dynamische Interaktionen zwischen den aufgetragenen Urothelzellen und den Matrixkomponenten zu unterstützen.

Die biologisch kompatible, kollagenhaltige Membran ist vorzugsweise eine intestinale Submukosa, azelluläre Harnblasen-Submukosa oder eine azelluläre dermale Matrix.

Besonders geeignete biologisch kompatible, kollagenhaltige Membranen sind
- SIS ("small intestinal submukosa"), die aus dem Dünndarm vom Schwein gewonnen wird, beinhaltet eine Kombination von unterschiedlichen Typen von Kollagen, Glykoproteinen, Proteoglykanen und funktionellen Wachstumsfaktoren, die eine Rolle bei der Zellmigration, dem Wachstum und der Differenzierung spielen (15). Die fast azelluläre, lyophilisierte Form dieser Membran (LSIS, "lyophilized small intestinal submukosa") ist von der Firma Cook Biotech (Mönchengladbach, Deutschland) erhältlich. Die nicht-lyophiliserte Form von SIS (NLSIS, nonlyophilized small intestinal submukosa), kann wie ABS (azelluläre Harnblasen-Submocosa) und ADM durch enzymatische und anschließende chemische Behandlung azellulär gewonnen werden, um ihre Immunogenität nach einer Transplantation des Konstruktes auf dieser Basis zu minimieren.
- ABS ("acellular bladder submukosa" oder azelluläres Harnblasen-Submukosa), die aus der Harnblasen-Submukosa vom Tier oder Mensch gewonnen wird, ist eine organ-spezifische Matrix, die eine physiologische Generierung des Harnblasengewebes vor und nach der Transplantation erlaubt.
- ADM (azelluläre dermale Matrix), die aus der Haut gewonnen wird, kann exklusiv aus patienteneigenem Gewebe präpariert werden und stellt somit kein Infektionsrisiko vom Donor zum Empfänger dar. Wird ADM gemeinsam mit patienteneigenem Serum verwendet, so wird ein vollständig autologes System erhalten, d.h. ein System ohne fetales Serum und ohne allogene bzw. xenogene Membran).

Das erfindungsgemäße Gewebetransplantatkonstrukt kann durch ein Verfahren erhalten werden, das folgende Schritte umfaßt:
(a) Aufbringen von organ-spezifischen Gewebezellen auf die biologisch kompatible, kollagenhaltige Membran;
(b) Proliferation der aufgebrachten organ-spezifischen Gewebezellen mittels stromaler Induktion in einem Kulturmedium unter Ausbildung einer oder mehrerer Schichten von organspezifischen Gewebezellen auf der Membran; und
(c) terminale Differenzierung der äußeren Schicht der expandierten organ-spezifischen Gewebezellen unter weiterer stromaler Induktion durch Verringerung mitogener Faktoren in dem Kulturmedium, wobei die Organ-spezifischen Gewebezellen Urothelzellen sind.

Ein Gewebetransplantatkonstrukt zur Rekonstruktion der Harnblase kann demnach durch ein Verfahren erhalten werden, das folgende Schritte umfaßt:
(a) Aufbringen von Urothelzellen auf die biologisch kompatible, kollagenhaltige Membran;
(b) Proliferation der aufgebrachten Urothelzellen mittels stromaler Induktion in einem Kulturmedium unter Ausbildung ein oder mehrerer Schichten von Urothelzellen auf der Membran; und
(c) terminale Differenzierung der äußeren Schicht der expandierten Urothelzellen unter weiterer stromaler Induktion durch Verringerung mitogener Faktoren in dem Kulturmedium.

Unter Verringerung mitogener Faktoren wird hier die Verringerung der Serenkonzentration (beispielsweise der Konzentration an FKS oder des autologen Serums) verstanden, während die Konzentration anderer stromaler nutritiver Stoffe konstant gehalten wird.

Die Schritte (b) und (c) werden durchgeführt, um die Gewebezellen, beispielsweise die Urothelzellen, unter Beibehalt des epithelialen Phänotyps zu expandieren, zu differenzieren und anschließend die äußere Schicht der Gewebezellen terminal zu differenzieren. Das Kulturmedium muß daher so gewählt werden, daß die gewählte Membran in Kooperation mit entsprechend adaptierten Kulturkonditionen zuerst die Migration und die Teilung und dann die funktionelle Reifung der kultivierten Zellen induzieren und somit wie im Fall der Rekonstruktion der Harnblase die Entwicklung eines hoch differenzierten, funktionierenden Urothelialkonstruktes ermöglichen.

Dazu sollten die in Schritt (a) auf die Membran aufgebrachten Gewebezellen zur Proliferation und Differenzierung sowie zur terminalen Differenzierung der äußeren Schicht in einem Kulturmedium kultiviert werden, das fötales Rinderserum (FKS) enthält. Besonders vorteilhaft ist es, die in Schritt (a) auf die Membran aufgebrachten Gewebezellen zur Proliferation und terminalen Differenzierung in einem Fibroblasten-konditionierten Medium (FBCM), das fötales Rinderserum (FKS) enthält, zu kultivieren.

Das Kulturmedium enthält vorzugsweise 5 bis 10 % FKS.

Im Anschluß an Schritt (b) sollte die Konzentration an FKS verringert werden, um nach der ausreichenden Expansion der Gewebezellen auf der Membran eine terminale Differenzierung der superfiziellen Zellen zu bewirken. Vorzugsweise wird die Konzentration an FKS zumindest um zumindest 50 % verringert, besonders bevorzugt wird die Konzentration an FKS von 5 bis 10 % auf 0 bis 1 % verringert.

Unter einer ausreichenden Expansion wird hierin verstanden, daß mehrere Schichten an Gewebezellen die Membran vollständig bedecken.

Zur Herstellung eines Gewebetransplantatkonstruktes zur medizinischen Anwendung umfaßt das Verfahren zur Herstellung eines Gewebetransplantatkonstruktes zur Rekonstruktion der Harnblase die Gewinnung von Urothelzellen aus kleinen Harnblasenbiopsien und deren (erste) Expansion unter konventionellen Bedingungen. Anschließend werden die Urothelzellen auf die biokompatiblen Membranen auf Kollagenbasis gebracht. Nach weiterer ausreichender Expansion durch stromale Induktion werden die Urothelzellen unter stromaler Induktion durch Erniedrigung von mitogenen Faktoren zur terminalen Differenzierung gebracht.

Die erfindungsgemäßen Gewebetransplantatkonstrukte sind Konstrukte, die aufgrund der terminalen Differenzierung der oberflächlichen Zellschicht dem in vivo Phänotyp entsprechen und somit in ihrer Funktion dem nativen Gewebe gleichen, im Falle einer Harnblase also dem nativen urothelialen Gewebe. Somit können beispielsweise erstmals funktionierende urotheliale Konstrukte gebildet werden, die einen geeigneten Schutz der biologischen Membran gegen Urin gewährleisten. Durch die Benutzung vom mit autologen Fibroblasten konditionierten Medien kann die Benutzung von xenogenen feeder layers sowie anderen kommerziell erhältlichen Zusätzen wie dem Wachstumsfaktor EGF vermieden werden. Das ist einerseits finanziell günstig, andererseits werden Bedenken hinsichtlich Toxizität, Kanzerogenität oder Krankheitsübertragung ausgeräumt. Anstelle von FKS kann autologes Serum verwendet werden, so daß in dem Kulturmedium keine xenogenen Zusätze enthalten sind.

Autologes Serum kann durch bekannte Verfahren aus dem Blut des zu behandelnden Patienten gewonnen werden.

Nachstehend wird die Erfindung anhand von Beispielen unter Bezugnahme auf die Zeichnungen näher erläutert. Es zeigt
- Fig. 1: eine graphische Darstellung der Methoden (1) bis (4) zur Proliferation und terminalen Differenzierung;
- Fig. 2: eine mittels Phasenkontrast-Mikroskopie erhaltene Aufnahme einer Monoschicht von porzinem Harnblasenmukosa;
- Fig. 3: eine DAPI-Anfärbung eines porzinen Harnblasen-Konstruktes auf LSIS (Fig. 3a: sichtbare Multischichten von Zellkernen auf der Membran; Fig. 3b und Fig. 3c: Mitoseaktivität sich teilender Kerne von kultivierten Zellen auf LSIS);
- Fig. 4: Paraffinschnitte von Urothelzellkonstrukturen auf azellulären Membranen ohne Fibroblasten-Induktion (Fig. 4A und Fig. 4B: Hämatoxylin-Eosin-Färbung auf ABS; Fig. 4C und Fig. 4D: Immunhistochemie mit PAN-Cytokeratin auf LSIS; Fig. 4E und Fig. 4F: *Hämatoxylin-Eosin-Färbung auf ADM*);
- Fig. 5: einen histologischen Vergleich von Urothelzellen, die auf azellulären Harnblasen-Submukosa anhaften und dort wuchsen (Fig. 5A: Urothelzellen, die ohne Fibroblasten-Induktion kultiviert wurden; Fig. 5B: Urothelzellen, die mit Fibroblasten-Induktion kultiviert wurden);
- Fig. 6: eine histologische, histochemische und immunohistochemische Analyse von porzinen und humanen Urothelzell-Konstrukten auf ABS (Figuren 6A, 6B und 6D: nach der Verringerung der FKS-Konzentration von 5 % auf 1 %; Fig. 6C und Fig. 6E: native porzine Harnblase; Fig. 6F: porzines Urothelzellen-Konstrukt und Fig. 6G: humanes Urothelzellen-Konstrukt);
- Fig. 7: eine histologische, histochemische und immunohistochemische Analyse von porzinen und humanen Urothelzell-Konstrukten auf LSIS (Fig. 7A: Hämatoxylin-Eosin-Färbung; Fig. 7B: AE1/AE3-Färbung; Fig. 7C: WGA-Färbung);
- Fig. 8: Paraffinschnitt von Urothelzell-Konstrukten auf NLSIS, wobei die FKS-Konzentration während der Kultivierung konstant gehalten wurde (Fig. 8A: 40fache Vergrößerung; Fig. 8B: 10fache Vergrößerung); und
- Fig. 9: eine schematische Darstellung eines erfindungsgemäßen Urothelzell-Konstruktes ist.

### Beispiele

Die folgenden Beispiele veranschaulichen das erfindungsgemäße Verfahren zur Herstellung von Harnblasen-Gewebetransplantatkontrukten.

Soweit nicht gesondert angegeben, haben die verwendeten Abkürzungen folgende Bedeutung:
- BCECF: 2',7'-Bis-(2-carboxyethyl)-5/6-carboxyfluorescein
- DAPI: 4'-6-Diamidino-2-phenylindol
- DMEM: Dulbecco's modifiziertes Eagle's Medium,
- ECM: extrazelluläre Matrix,
- EGF: Epidermaler Wachstumsfaktor
- FB: Fibroblast
- FKS: fötales Rinderserum
- KGM: Keratinozyten-Wachstumsmedium
- UC: Urothelzellen
- PBS: Phosphat-gepufferte physiologische Kochsalzlösung
- PI: Propidiumiodid,
- WGA: Weizenkeim-Agglutinin
- FBCM: Fibroblasten-konditioniertes Medium.

### Materialien und Methoden

*Biologische azelluläre Membranen*: Lyophilisierte kleine Darmsubmukosa (LSIS) ist von Cook Biotech (Mönchengladbach, Deutschland) kommerziell erhältlich. Zur Herstellung von nicht-lyophilisierter kleiner Darmsubmukosa (NLSIS), azellulärer Blasensubmukosa (ABS) und zellulärer dermaler Matrix (ADM) wurden Schweineproben mit 1 % TritonX-100 (Sigma-Aldrich, Taufkirchen, Deutschland) 48 h behandelt.

Die Membranen haben jeweils eine Größe von bis zu 40 cm².

*Urothel- und Fibroblastenzellkulturen*: Segmente aus der Schweineblase wurden aus acht getöteten Tieren erhalten. Schweineproben sind aufgrund ihrer Ähnlichkeit zu der humanen Blase ausgewählt worden [15]. Zusätzlich wurden humane Gewebeproben aus der Harnblase (n = 4) zusätzlich aus erwachsenen Patienten, die sich der Blasenchirurgie unterziehen mußten, erhalten.

Die mukosalen und submukosalen Gewebe wurden getrennt zerkleinert, mit Kollagenase B (200 u/ml) (Worthington Biochemical, Lakewood, NJ, USA) verdaut, durch Zentrifugation (2000 U/min) gesammelt und in einer feuchten Atmosphäre aus 95 % Luft und 5 % Kohlendioxid kultiviert. Die Urothelzellen und die Fibroblasten wurden jeweils in dem supplementierten Keratinocytwachstumsmedium (KGM) (CellSystems Biotechnologie, St. Katharinen, Deutschland) bzw. Dulbecco's modifiziertem Eagle's Medium (DMEM) (Invitrogen, Karlsruhe, Deutschland), versetzt mit 5 bis 10 % FKS, kultiviert. Immuncytochemische Analysen mit AE1/AE3 (1 : 150 Konzentration), Vimentin (1 : 100) und α- Actin des Glattmuskels (1 : 100) (Dako, Glastrup, Dänemark) wurden durchgeführt.

Definierte Zahlen der Urothelzellen wurden auf die Oberfläche der Membranschichten (10⁵ Urothelzellen/cm²) aufgetragen und zweimal wöchentlich für bis zu 28 Tage mit einer der folgenden Methoden (1) bis (4) behandelt:
(1) Die Urothelzellen wurden mit DMEM, supplementiert mit 10 % FKS, versetzt (Gruppe 1). Gruppe 1 ist eine Kontrollgruppe;
(2) Die Urothelzellen wurden mit serumfreiem Medium, supplementiert mit epidermalem Wachstumsfaktor (EGF), Rinderhypophysenextrakt (BPE), Hydrokortison, Transferrin, Insulin und Epinephrin, in der vom Hersteller empfohlenen Konzentration supplementiert (Gruppe 2). Gruppe 2 ist eine Kontrollgruppe;
(3) Die Fibroblaten wurden in DMEM mit 5 % FKS auf dem Boden eines Transwellsystems co-kultiviert(Gruppe 3). Gruppe 3 ist eine Kontrollgruppe;
(4) Urothelzellen wurden mit Fibroblasten-konditioniertem Medium (FBCM), supplementiert mit FKS (5 % für die ersten 14 Tage und 1 % für die folgenden zwei Wochen) kultiviert (Gruppe 4).

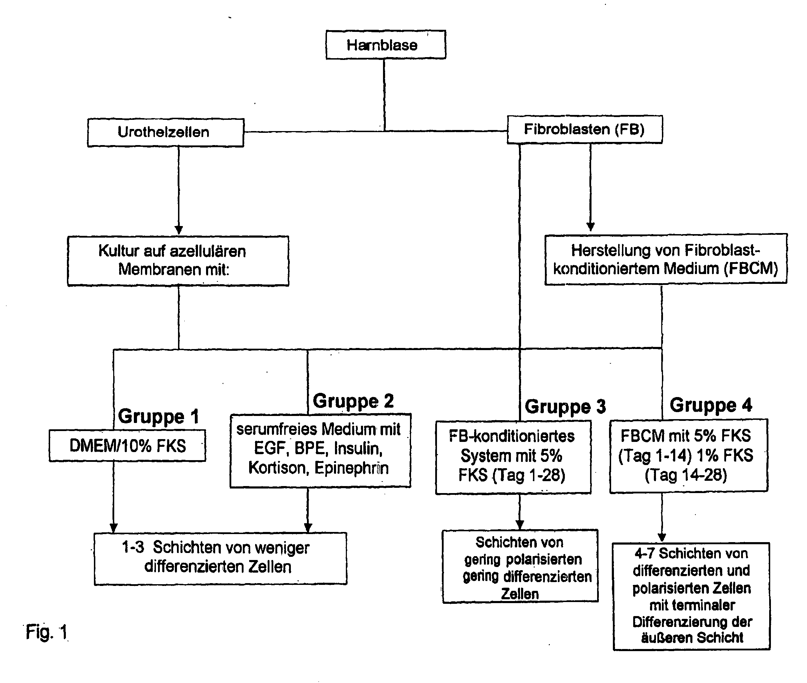

Fig. 1 ist eine graphische Darstellung der Methoden (1) bis (4). Die Urothelzellen und Fibroblasten wurden von einer Harnblase mittels enzymatischer Behandlung geerntet. Nach einer (ersten) Expansion wurden die Urothelzellen auf den angegebenen Membranen unter Verwendung der Kulturmedien gemäß den Methoden (1) bis (4) kultiviert. Die Fibroblasten-konditionierten Kultursysteme (Gruppen 3 und 4) zeigten eine höhere Anzahl von Zellschichten im Vergleich zu nicht Fibroblasten-konditionierten Systemen (Gruppen 1 und 2). Die Verringerung mitogener Stimuli nach Tag 14 in Gruppe 4 induzierte im gleichen Zeitraum einen hohen Differenzierungsgrad der Urothelzellen.

Alle in den Methoden (1) bis (4) verwendeten Medien wurden zunächst mit 0,09 mM Calcium supplementiert. Zur Schichtenbildung wurden die Kulturen auf die Luft-Flüssigkeit-Grenzfläche unter verstärkter Calciumkonzentration (2,5 mM) gebracht.

*Harnblasen-Fibroblasten-konditioniertes Medium:* Nach der primären Kultur wurden 10⁶ Fibroblasten in eine 75 cm² Zellkultur-Flasche überführt und mit DMEM, supplementiert mit 5 % oder 1 % FKS, versetzt. Bei 90 % Konfluenz wurden die Medien gewechselt und 24 h später gesammelt, filtriert (Falcon, BD, Heidelberg, Deutschland) und bei 4 °C gelagert.

### Zelluläre Adhäsion, Proliferation und Lebensfähigkeit

Um die Oberflächendichte der kultivierten Zellen auf den Membranen zu bestimmen, wurde die Zellzahl nach der Abtrennung von der Kulturschale von der Zellzahl in der Suspension 2 und 10 h nach der Saat auf die Membran subtrahiert. Proliferationsassays wurden mit 4'-6-Diamidino-2-phenylindol (DAPI) an den Tagen 2, 14 und 28 nach der Anlagerung durchgeführt. Lebensfähigkeitsassays wurden 6 h, 14 Tage und 28 Tage mit BCECF (2',7'-Bis-2-carboxyethyl-5- und -6-carboxyflureszein) und PI (Propidiumiodid) (beide von Mobitec, Göttingen, Deutschland) (5 µmol jeweils, 20 min Inkubation) durchgeführt.

### Histologisches, histochemisches und immunohistochemisches Anfärben von kultivierten Zellen auf Membranen

Die Materialien wurden in 4 % neutral gepuffertem Formaldehyd für 24 h fixiert und in Paraffin eingebettet. Die Sektionen wurden entweder mit Hematoxylin-Eosin eingefärbt oder zur Immunohistochemie und Lektinverfärbung mit Weizenkeimagglutinin (WGA) verwendet. Die Verdünnung des primären Antikörpers für Pankytokeratin (AE1/AE3, Dako) betrug 1 : 150 nach der Vorbehandlung der Präparate mit Pronase. Der Nachweis des primären Antikörpers wurde unter Verwendung von entweder einem Meerrettichperoxidase- (LASB-System von Dako) oder einem alkalischen Phosphatasenachweissystem (Vectastain ABC-Kit, Vector Laboratories, Grünberg, Deutschland) durchgeführt. Daher war das Farbsubstrat Diaminobenzidin (braune Farbe) oder alkalisches Phosphataseneufuchsin (rote Farbe). Die Verdünnung des Lektin WGA (Vector Laboratories) betrug 1 : 100. Die weitere Behandlung wurde mit Vectastain ABS-Kit durchgeführt. Die Immunfärbung mit Anti-Uroplakin III Antikörper (erhältlich von Progen, Heidelberg, Deutschland) wurde bei einer Verdünnung von 1 : 10 durchgeführt und wurde mit LSAB-Kit (Dako) gemäß den Instruktionen des Herstellers entwickelt.

### Ergebnisse

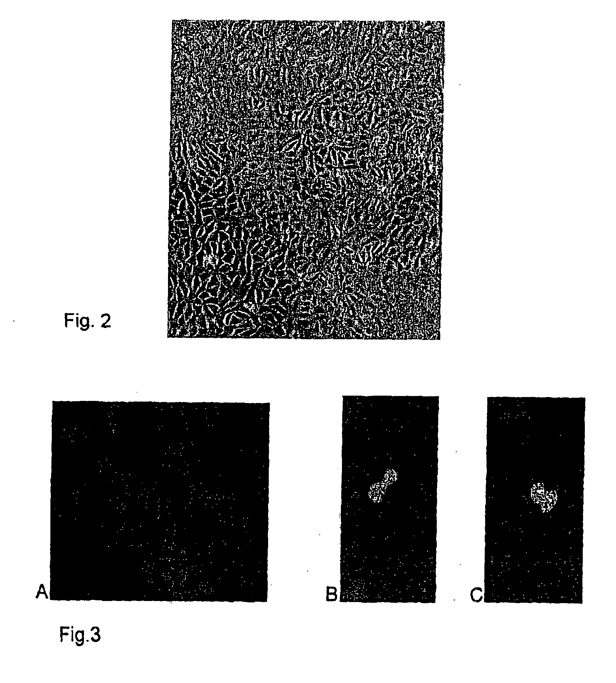

*Konventionelle Zellkulturen*: Primäre Kulturen von Fibroblasten und Urothelzellen wurden aus kleinen Biopsien von porzinen und humanen Harnblasen geerntet (Fig. 2). Urothelzellen - Anfärbung mit breit reagierenden Anti-Cytokeratin-Antikörpern bestätigt ihren epithelilaen Charakter. Fibroblasten färbten positiv mit Vimentin und reagierte nicht mit PAN-Cytokeratin-Antikörpern. Beide Zelltypen zeigten negative Markierung hinsichtlich des α-Aktins des glatten Muskels.

Fig. 2 ist eine mittels Phasenkontrastmikroskopie (10fach) erhaltene Aufnahme von porzinen Urothelzellen, die von porzinen Harnblasenmukosa mittels Mikrodissektion geerntet wurden. In dem *zweidimensionalen* serumfreien Kultursystem zeigten die Zellen ihren polygonalen Phänotyp.

Das stark supplementierten Kulturmedium KGM ist als ein gutes Medium zum Aufrechterhalten des Uroethialproliferationspotentials *in vitro* bekannt. Jedoch könnte sein hoher Gehalt an Wachstumsfaktoren und Zusätzen Schwierigkeiten bei der Bestimmung der Regulationsfaktoren mit Fibroblasten-Ursprung an Urothelzellen verursachen. Deshalb wurde zusätzlich zu diesem Medium (Gruppe 2) ein universelles Medium (DMEM/FKS) in anderen Experimenten zur besseren Bestimmung der Urothelial/ECM-Wechselbeziehungen (Gruppe 1) und Urothelial/FB/ECM-Wechselbeziehungen durch indirekte Co-Kulturen (Gruppe 3) und FBCM-Kultureinstellung (Gruppe 4) verwendet.

*Zellhaftung an die Membranen*: Die Zellanhaftung betrug 80 % auf ABS und NLSIS und 70 % auf LSIS und 60% auf ADM 2 h nach der Saat. Nach 10 h offenbart die Endzahl an Zellen in der Suspension, daß keine restlichen Zellen in der Lösung aus NLSIS und ABS und 10 % an Zellen in der Lösung aus LSIS und 20% in der Lösung aus ADM verblieben waren. Die Kultur von porzinen und humanen Urothelzellen mit nicht-konditionierten Medien und ohne Zusatz von FKS und Wachstumsfaktoren zeigte nur schlechte Anhaftung an Zellen, da diese innerhalb der ersten Tage (Daten nicht gezeigt) starben und daher nicht weiter berücksichtigt wurden.

*Zellebensfähigkeit*: Die Zellebensfähigkeit blieb während der Periode von 28 Tagen in allen Gruppen hoch (≥ 80 %). Nach Anwendung der Doppelfärbung auf die Urothelzellen-Kulturen offenbarte grüne Fluoreszenz von metabolisierten BCECF-AM durch die Zellen. Urothelzellen, die an der Oberfläche der Membranen angelagert sind, überlebten und hielten ihre Lebensfähigkeit nach 6 h aufrecht. Sie bildeten zusammenfließende Schichten auf der gesamten Oberfläche der Membranen an den Tagen 14 und 28. Es wurden fast keine toten Zellen (< 5%) bis zu Tag 14 in allen Gruppen nachgewiesen. Im Vergleich zu den Kontrollgruppen (Gruppen 1 und 2) zeigten porzine und humane Urothelzellen unter Fibroblasten-Induktion eine höhere Zahl an lebensfähigen Zellen, was intensivere grüne Fluoreszenz von mehreren Schichten (Daten nicht gezeigt) wiedergibt. In Gruppe 4 waren weniger als 10 % der Zellen am Tag 28 tot, während in Gruppe 3 eine höherer Anteil an toten Zellen auf den Membranen (20 %) nachweisbar war.

*Zellproliferation*: Zellkerne, angefärbt mit DAPI, zeigten unterschiedliche Mitosestufen ohne nachweisbaren apoptotischen Anteil (<5 %) an den Tagen 2 und 14 (Fig. 3) in allen Gruppen. Mehrschichten von Kernen waren deutlich reicher in Kulturen mit Fibroblasten-Induktion bis zu Tag 28 (Gruppen 3 und 4). Die Mitose-Indices in Kulturen erhöhten sich um das 1,7fache (porzine Zellen) und 1,3fache (humane Zellen) in den Gruppen 1 und 2 (Kontrollgruppen) und um das 3,2fache (porzine Zellen) und 2,9fache (humane Zellen) in den Gruppen 3 und 4 von Tag 2 bis 14. In Gruppe 4 wurden fast keine proliferativen Kerne am Tage 28 nachgewiesen, während Kerne in unterschiedlichen Mitosestufen in Gruppe 3 noch nachweisbar waren.

Fig. 3 zeigt eine DAPI-Anfärbung (40fache Vergrößerung) eines porzinen Harnblasen-Konstruktes auf LSIS. Die Zellkerne wurden durch indirekte Immunfluoreszenz am Tag 14 nach der Saat detektiert. Multischichten von Zellkernen sind sichtbar (Fig. 3A) auf der Membran. Die Mitoseaktivität der sich teilenden Kerne der kultivierten Zellen auf LSIS wird in Fig. 3B und Fig. 3C (fluoreszenzmikroskopische Aufnahme, 40fache Vergrößerung) gezeigt.

*Histologische, histochemische und immunohistochemische Analysen der Konstrukte:* Die Analyse von konfluierenden Kulturen zeigte positive Zellen für PAN-Cytokeratin-Antikörper. Außerdem wurden Anfärbungen mit WGA und Anti-uroplakin III Antikörper in nativem Gewebe und in porzinen und humanen Urothelzell-Konstrukten durchgeführt.

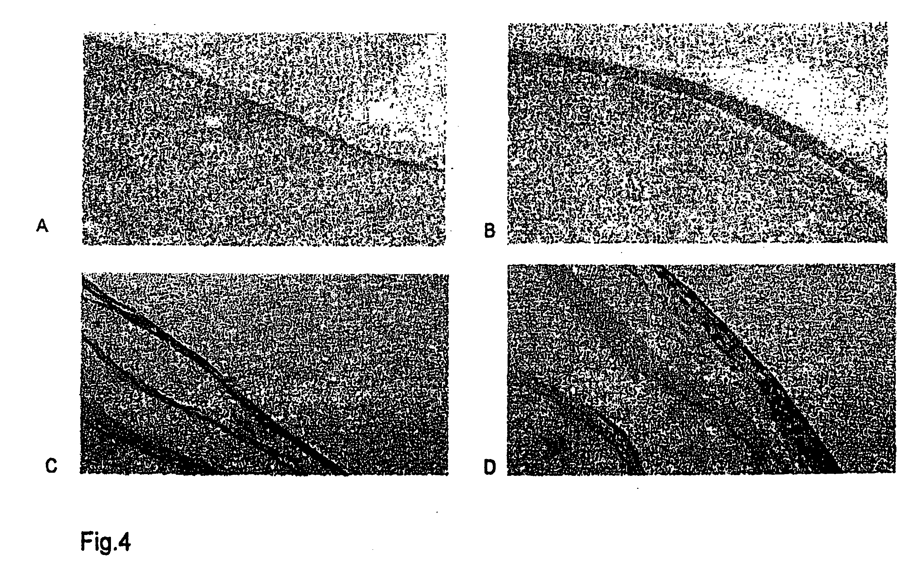

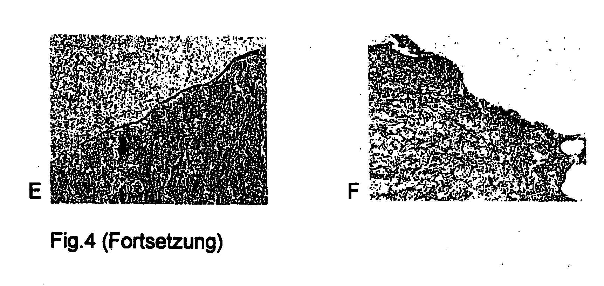

Die histologischen Analysen zeigten die Fähigkeit von Urothelzellen, auf allen vier Membrantypen anzulagern, zu migrieren und zu proliferieren. In den Gruppen 1 und 2 (Kontrollgruppen) migrierten die Zellen an Tag 1 aus kleinen Gruppen zu 1 bis 2 Schichten, was die gesamte Oberfläche der Membranen an Tag 28 auf LSIS bzw. zu 2 bis 3 Schichten auf NLSIS und ABS zunehmend bedeckt (Fig. 4). In einem anderen Versuch wurden Urothelzellen auf Membranen co-kultiviert, aber von Fibroblasten durch eine Transwell-Kultureinlage abgetrennt (Gruppe 3). In der dritten Versuchsreihe wurden Epithelzellen auf den Membranen mit Medien, konditioniert durch Fibroblasten (Gruppe 4), kultiviert. Urothelzellen, kultiviert in Medien, die 10 % FKS (Gruppe 1) oder EGF und andere Standardzusätze (Gruppe 2) enthielten, zeigten begrenzte Migration und Membranabdeckung, schlechtere Wachstumseigenschaften, geringere Proliferationsraten und wenig Differenzierung im Vergleich zu den Kulturen mit Fibroblasten-Induktion. Sie zeigten ebenso allmähliche Alterung nach 4 Wochen. Weder FKS noch EGF und andere exogene Zusätze induzierten eine Schichtenbildung noch eine terminale Differenzierung von porzinen und humanen Urothelzellen (Gruppen 1 und 2) unter den vorgegebenen Bedingungen.

Fig. 4 zeigt Paraffinschnitte (40fache Vergrößerung) von Urothelzellkonstrukturen auf azellulären Membranen ohne Fibroblasten-Induktion (Figuren 4A, 4B, 4E und 4F: Hämatoxylin-Eosin-Färbung ; Fig. 4C und Fig. 4D: Immunhistochemie mit PAN-Cytokeratin). Die Urothelzellen auf ABS (Fig. 4A), LSIS (Fig. 4C) und ADM (Fig. 4E) bildeten eine Monoschicht von flachen Zellen am Tag 1 nach der Saat. Die Zellen setzen die Proliferation fort und zeigten nach einem Zeitraum von 28 Tagen eine eher würfelförmige Morphologie auf ABS (Fig. 4 B), LSIS (Fig. 4D) und ADM (Fig. 4F). Die Kulturen bestanden am Tag 28 aus zwei bis drei Schichten von Zellen auf ABS (Fig. 4B) und ein bis zwei Schichten auf LSIS (Fig. 4D)und ADM (Fig.4F). Die Immunanfärbung des rekonstruierten Urothels in vitro mit PAN-Cytokreatin auf LSIS (Fig. 4C und 4D) bestätigten den epithelialen Ursprung der Urothelzellen.

Eine Optimierung des Kulturmediums wurde mittels löslichen Nährstoffen, abgesondert von Fibroblasten (Gruppen 3 und 4), erreicht. Eine Kultur von Urothelzellen auf den Matrices mit Fibroblasten, abgetrennt durch die Transwellkultureinlage, ergab ähnliche Ergebnisse hinsichtlich der Urothelzellen-Morphologie, Schichtenbildung und Differenzierung im Vergleich zu Experimenten mit FBCM.

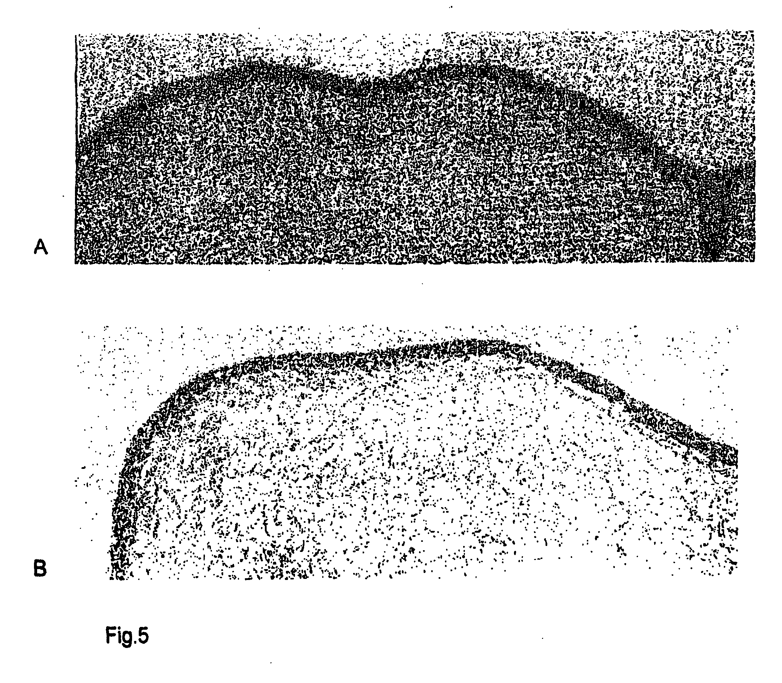

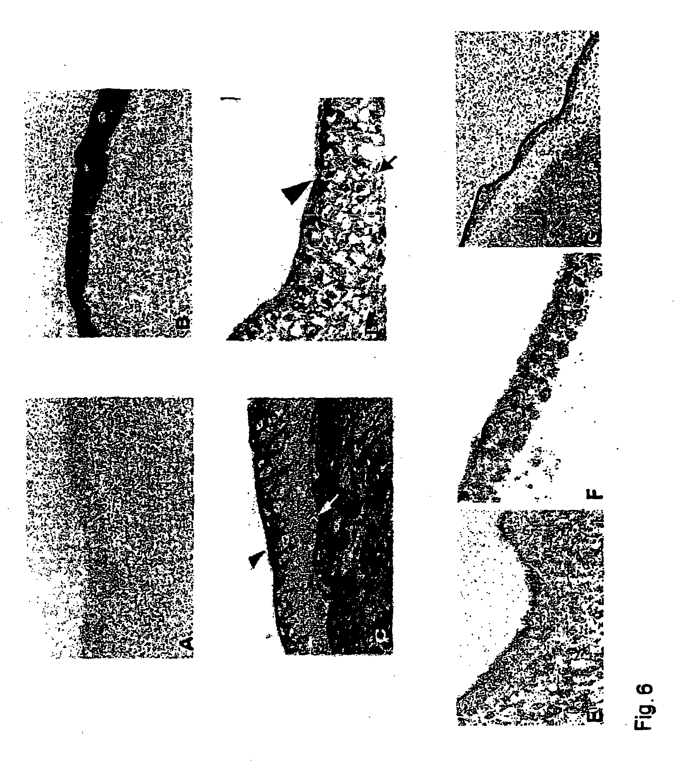

Diese zwei Gruppen von Experimenten (Gruppen 3 und 4) zeigten eine erhöhte Zahl von Urothelzell-Schichten auf den Membranoberflächen in demselben Zeitraum im Vergleich zu Urothelzell-Kulturen ohne Fibroblasten-Induktion (Gruppen 1 und 2) (Fig. 5). Tatsächlich offenbarten Hematoxylin- und Eosinanfärbungen eine zunehmende Migration von Urothelzellen aus kleinen Gruppen zu ein bis drei Schichten (Fig. 3) gegenüber drei bis sieben (Gruppe 4) oder mehr (Gruppe 3) Zellschichten (Fig. 6 bis 8), die die vollständigen Membranoberflächen mit Größen bis zu 40 cm² an Tag 28 abdecken.

Fig. 5 zeigt in jeweils 40facher Vergrößerung einen histologischer Vergleich von Urothelzellen, die auf azellulären Harnblasen-Submukosa (ABS) anhaften und dort wuchsen. Die Zellen wurden ohne Fibroblasten-Induktion (nichtkonditioniertes Medium) (Fig. 5A) und mit Fiblorblasten-Induktion (konditioniertes Medium) (Fig. 5B) kultiviert. Unter einer relativ geringen Calciumkonzentration (0,09 mM) bedecken die Zellen die Membran 5 Tage nach der Saat mit 1 bis 2 Schichten in dem nicht-konditionierten System (Fig. 5A), verglichen mit 2 bis 3 Schichten der Zellen (Fig. 5B) mit Fibroblasten-Induktion.

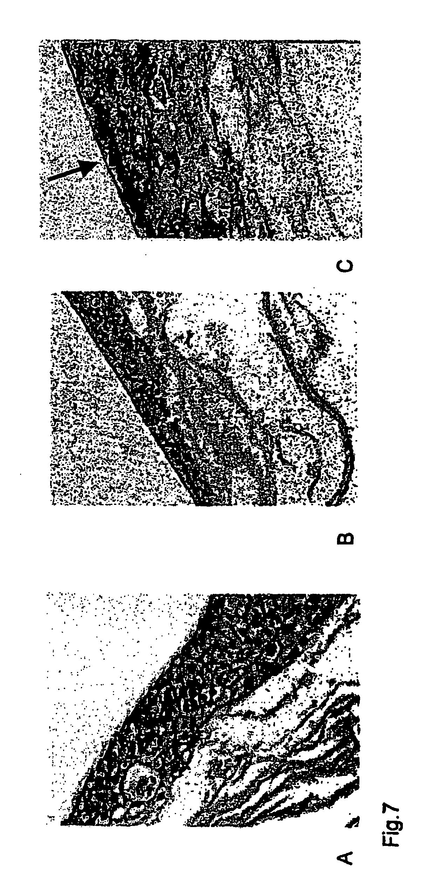

Nach der beschleunigten Proliferation durch Fibroblasten-Induktion in Gruppen 3 und 4 (Tage 14) erreichten die Zellen die terminale Reifung unter hoher Calciumkonzentration (2,5 mM) nicht. Um die terminale Differenzierung zu induzieren, wurde die Konzentration von FKS von 5 % auf 1 % nach der Proliferationsphase (14 Tage) in Gruppe 4 verringert. Dies reduziert die Konzentration an mitogenen Faktoren, während die Konzentration an anderen Nährstoffkomponenten kontant blieb, und regulierte daher den proliferativen Zustand der Zellen nach unten. Unter diesen Bedingungen fand die vollständige Differenzierung der oberflächlichen porzinen und humanen Zellen statt, wie in den histologischen, histochemischen und immunohistochemischen Analysen von Fig. 6 und Fig. 7 gezeigt wird. Dieser Zustand wurde bis zu Tag 28 aufrechterhalten. Die intensive Färbung der oberflächlichen Zellen in Urothelkonstrukten mit WGA, zusätzlich zu deren starker Expression von Uroplakin III, was ein bezeichnender Marker für die Urotheldifferenzierung ist, zeigen ebenso die terminale Differenzierung dieser Zellen (Fig. 6 und 7).

Fig. 6 zeigt eine histologische (Fig. 6A), histochemische (Fig. 6C, 6D) und immunhistochemische (Fig. &B, 6E, 6F, 6G) Analyse von porzinen und humanen Urothelzell-Konstrukten auf ABS (Figuren 6A, 6B und 6D) nach Verringerung der FCS-Konzentration von 5 % auf 1 % im Vergleich zu nativer porziner Harnblase (Fig. 6C, Fig. 6E). Die Hämatoxylin-Eosin-Färbung (Fig. 6A) von Urothelzellen, die auf ABS kultiviert wurden, zeigt ein geschichtetes Neo-Urothelium am Tag 28 nach der Saat. Die kultivierten Urothelzellen zeigen eine stark positive (dunkelbraune Färbung mit Farbsubstrat Diaminobenzidin) Reaktion mit einem Antikörper gegen PAN-Cytoceratin (Fig. 6B). Die WGA-Färbung des Urothelzell-Konstrukts (Fig. 6D) ist dem nativen Urothelium (Fig. 6C) bei Fibroblasten-Induktion sehr ähnlich. WGA markiert überwiegend die hoch differenzierten Schichten des nativen Urotheliums (Fig. 6C) (Pfeilkopf) und schließt die Oberflächenschicht des Urothelzell-Konstrukts in vitro (Fig. 6D) (Pfeilkopf) ein. Die weniger differenzierten Zellen werden durch WGA nicht gefärbt (Pfeile). Die Färbung mit Anti-Uroplakin III-Antikörpern (braune Farbe) in nativen porzinen Urothelium (Fig. 6E) und den porzinen (Fig. 6F) und humanen (Fig. 6G) Urothelzell-Konstrukten zeigt eine starke Ähnlichkeit miteinander. Wie bereits gezeigt, ist die intensive Expression von Uroplakin III ausschließlich in der Oberflächenschicht des nativen Urotheliums (Fig. 6E) wie auch ausschließlich in den Oberflächenschichten der Urothelzell-Konstrukte (Fig. 6F und Fig.6G) zu sehen (40fache Vergrößerung in den Figuren 6A bis 6F und 10fache Vergrößerung in Fig. 6G).

Fig. 7 zeigt eine histologische, histochemische und immunhistochemische Analyse von Urothelzell-Konstrukten auf LSIS mit Fibroblasten-Induktion am Tag 28. Nach Verringerung der FKS-Konzentration von 5 % auf 1 %, zeigt die Hämatoxylin-Eosin-Färbung bis zu sieben Schichten von morphologisch differenzierten Zellen (Fig. 7A). Die positive Färbung der Urothelzellen mit AE1/AE3 bestätigt deren epithelialen Ursprung (Fig. 7B). Die Färbung mit WGA (Fig. 7C) zeigt, daß sich die Oberflächenzellen der Urothelzell-Konstrukte am intensivsten mit Lectin (Pfeil) färben im Vergleich zu den weniger differenzierten Zellen in den unteren Schichten (Fig. 7B: 10fache Vergrößerung, 40fache Vergrößerung in Fig. 7A und Fig. 7C).

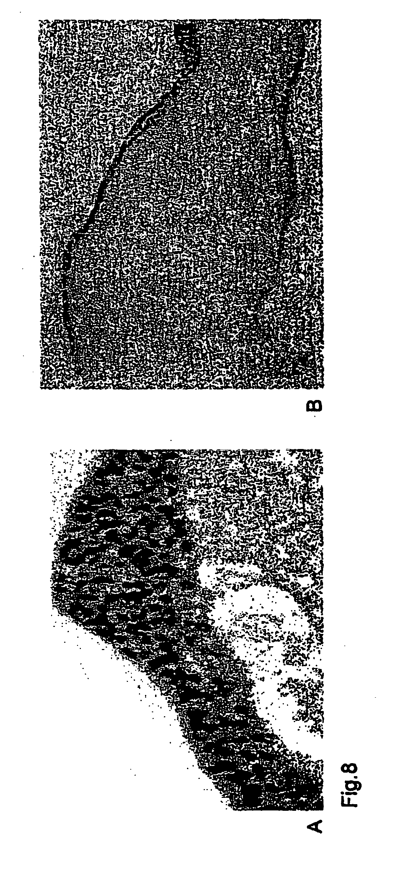

Die Verringerung von FKS, EGF und BPE in den Gruppen mit nicht-konditioniertem Medium (Gruppen 1 und 2) führte zu nicht-proliferierenden Zellen, die allmählich abstarben. In Gruppe 3 wurde die FKS-Konzentration 28 Tage bei 5 % gehalten. Die Migrations- und Proliferationsphase wurden aufrechterhalten, was zur Expansion von Zellen auf die andere Seite der Membran, Schichtenbildung von morphologisch weniger polarisierten und schwach differenzierten Zellen (Fig. 8), führte. Um die unterschiedlichen Proliferationsphasen des Neourotheliums in Gruppe 4 zu bestätigen, zeigte die Demonstration der Aktivität der Zellkerne mittels DAPI-Anfärbung meistens mitotische Kerne in der Proliferationsphase der Kultur (Tag 14, FKS 5 %), während in dem terminal differenzierten Urothelium (Tag 28, FKS 1 %) sich die Menge an teilenden Kernen signifikant verringerte.

Fig. 8 zeigt einen Parrafinschnitt von Urothelzell-Konstrukten auf NLSIS. In dem hoch proliferierten Kultursystem wurde die FKS-Konzentration während eines Zeitraumes von 28 Tagen unter Fibroblasten-Induktion konstant gehalten (5 %). In diesem Kultursystem blieben die Zellen proliferativ und zeigten einen Verlust an Differenzierung. Sie teilten sich weiter und migrierten zu der anderen Seite der Membran, so dass die NLSIS mit vielen Schichten aus nichtpolarisierten Zellen bedeckte wurde und somit das Bild eines hyperproliferativen Urotheliums zeigte (40fache Vergrößerung in Fig. 8A, 10fache Vergrößerung in Fig. 8B).

### Bewertung der Ergebnisse

Kleine Darmsubmukosa, azelluläre dermale Matrix und ABS unterstützten das Wachstum von Urothelzellen, was Zell/ECM-Wechselbeziehungen und die Erzeugung von 2 bis 3 Schichten von Urothelzellen in stark supplementierten Kulturbedingungen (Gruppen 1 und 2) ermöglichte. Sowohl ABS als auch NLSIS waren nahezu äquivalent in ihrer Fähigkeit, die Urothelproliferation und -differenzierung zu unterstützen. Der kleine Unterschied zwischen LSIS und nicht-lyophilisierten Membranen kann sich aus lyophilisierten Form von LSIS ergeben [18]. ADM zeigte ebenfalls gute Urothel-Proliferation und - Differenzierung.

Es ist berichtet worden, daß serumfreies Medium mit Wachstums-stimulierenden Hilfsmitteln kritisch für das Urothelzellen-Wachstum unter konventionellen Kulturbedingungen ist [19]. Die Supplementation von exogenen Faktoren oder 10 % FKS in Kontrollgruppen zeigte mäßige induktive Wirkungen auf Urothelzellen, kultiviert auf den Membranen (Gruppen 1 und 2).

Um die Kulturbedingungen von Urothelzellen weiter zu verbessern, wurde die induktive Wirkung von Fibroblasten aus der *lamina propria* auf Urothelzellen genutzt, wodurch diese mit potentiellen Wachstumsfaktoren versorgt werden (Gruppen 3 und 4). Dies führte zur Migration der porzinen und humanen Urothelzellen mit vollständiger Bedeckung der bis zu 40 cm² großen Membranen und einer höheren Proliferation in einem kürzeren Zeitraum ohne spontane noeplastische Tranformation. Die Stimulation wurde nur durch 5 % FKS-Supplementation unter Fibroblasten-Induktion (Gruppen 3 und 4) ermöglicht und war signifikant höher als mit exogen vervollständigten Medien mit 10 % FKS (Gruppe 1) oder EGF und allen anderen Zusätzen (Gruppe 2).

Dies zeigt, daß Medien mit Fibroblastenkomponenten aktive Wachstumsfaktoren enthalten, die in ausreichenden Konzentrationen abgesondert werden, um die niedrige FKS-Konzentration (5 %) oder die Abwesenheit von EGF, BPE, transferrin, Hydrokortison und Insulin, die zu den traditionell hoch supplementierten Medien zugegeben wurden, zu kompensieren. Diese Ergebnisse zeigen ebenso, daß die mitogene Wirkung und der Signalmechanismus vom ECM-Gehalt von biologischen Membranen und Fibroblasten-induktiven Faktoren miteinander synergistisch sind. Dieses günstige Phänomen kann in optimierten Strategien verwendet werden, um Urothelium auf biologischen Matrices in vitro zu proliferieren. Außerdem minimiert die Verwendung von autologen Fibroblasten (anstelle von xenogenen feeder layers, d. h. als Wachstumsunterlage in der Zellkultur dienende xenogene Zellschichten) das Risiko der Übertragung von Infektionen.

Die Erhöhung der Mitoserate und die verzögerte Reifung von Urothelzellen wurde in dieser Studie unter 5 % FKS-Supplementation in dem ersten Teil der Urothelialtechnik erreicht. Weitere Differenzierung trat nach der Reduktion von FKS (Gruppe 4) auf. Tatsächlich inhibierte die FKS-Konzentration eine verzögerte Proliferation der Zellen und schaltete die proliferative Phase zu der Differenzierungsphase des Urotheliums unter stromaler Regulierung und in Abwesenheit von anderen exogen zugegebenen Wachstumsfaktoren. Die Zellproliferation wurde dann herunterreguliert, was die terminale Differenzierung ermöglichte. Dies kann von großer Wichtigkeit für die Funktionalität des Urotheliums und der Verhinderung von Hyperproliferation von Zellen nach der Implantation sein. Alle vier Membranen zeigten die terminal differenzierten Zellen nur in der oberflächlichen Schicht, so daß sie dem nativen Gewebe ähnelten.

Im Vergleich zu Fibroblasten-konditionierten Kulturen von Urothelzellen auf Kollagengel [10,15] zeigte sich somit, daß die Assoziation von zusätzlichen Komplex-ECM-Komponenten, die in den Membranen enthalten sind, und das schrittweise angepaßte Medium die Kulturbedingungen auf diesen Matrices verbesserte. Tatsächlich konnten die Urothelzell-Kulturen auf dem Kollagengel weder in mehr als 3 Schichten proliferiert werden noch eine terminale Differenzierung mit FBCM [10] erreichen und zeigten keine zelluläre Expression für Uroplakin III [15].

Zusätzlich zu statischen Kulturen kann das in Methode (4) vorgeschlagene Kulturmedium für Kulturen in Perfusionskammern und Bioreaktoren verwendet werden, wenn unterschiedliche Medien für spezielle Zelltypen verwendet werden, die auf beiden Seiten der Membran kultiviert werden. Ähnliche Studien für die Induktion von Harnblasen-Muskelzellen würden hilfreich zur Konstruktion von komplexen lebenden Harnblasentransplantaten mit hoch differenzierten Zellen, bestehend aus Urothelzellen auf einer Seite und glatten Harnblasen-Muskelzellen auf der anderen Seite der Membran sein. Hier ist jedoch von Bedeutung das die terminal differenzierten Urothelzellen das Transplationsmaterial, d. h. die Membran (mit oder ohne Muskelzellen) gegen Urin schützen.

Im Vergleich zu Urothelzellen, die in Medien mit relativ teuren Zusätzen kultiviert wurden, ermöglicht das erfindungsgemäße Verfahren die Proliferation und terminale Differenzierung von Urothelzellen in einem kürzeren Zeitraum unter Verwendung von Medien, die keine der genannten, relativ teuren Supplemente enthalten. Dieser günstige finanzielle Aspekt ist ein interessanter Faktor, der bei der Herstellung von Harnblasen-Transplanten berücksichtigt werden sollte.

Die induktive Wirkung von FBCM wurde nicht nur an porzinen Urothelzellen beobachtet, sondern ebenso an humanen Urothelzellen (Fig. 6).

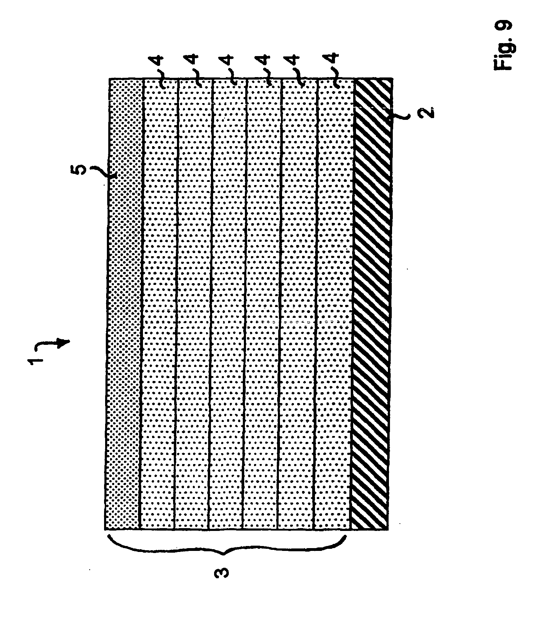

Fig. 9 zeigt eine schematische Darstellung des erfindungsgemäßen Gewebetransplantatkonstruktes 1. Auf der Membran 2 befinden sich sieben Urothelzell-Schichten 3, wobei die äußere Schicht 5 eine Schicht aus terminal differenzierten Urothelzellen ist. Die Schichten 4 bestehen aus nicht terminal differenzierten Urothelzell-Schichten.

Die erfindungsgemäßen Kulturbedingungen von Urothelzellen, weisen therapeutisches Potential zur Induktion der Urothelerzeugung aus einem erkrankten Organ auf.

Durch das Wissen, daß die Barriereeigenschaften von stark differenzierten Urothelkonstruktionen dieselben sind, wie die des nativen Urotheliums [20], kann das erfindungsgemäße Kulturverfahren helfen, näher an das ultimative Ziel der Erzeugung von lebenden und funktionell autologen Transplantaten mit ähnlichen Qualitäten als die native Blase zu kommen.

Zur Herstellung des erfindungsgemäßen Konstruktes und zur Ausführung des erfindungsgemäßen Verfahrens ist die Supplementierung teurer Wachstumsfaktoren nicht erforderlich, was einen erheblichen finanziellen Vorteil darstellt. Im Vergleich zu ähnlichen Konstrukten kann mit dem erfindungsgemäßen Verfahren das erfindungsgemäße Konstrukt in wesentlich kürzer Zeit erhalten werden. Die Behandlungsdauer eines Patienten, dessen Harnblase rekonstruiert werden muß, kann somit erheblich verkürzt werden. Durch das Vermeiden der Benutzung der Gastrointestinalsegmente können assoziierte Komplikationen verhindert und somit weitere Kosten eingespart werden.

### Literatur

1 Falke G, Caffaratti J, Atala A. Tissue engineering of the bladder. World J Urol.2000; 18:36-43.
2 Atala A. New methods of bladder augmentation. BJU Int 2000; 85:24-34
3 Atala A. Bladder regeneration by tissue engineering. BJU Int 2001; 88:765-70
4 Yoo JJ, Meng J, Oberpenning F, Atala A. Bladder augmentation using allogenic bladder submukosa seeded with cells. Urology 1998; 51:221-5
5 Zhang Y, Kropp BP, Moore P, et al. Coculture of bladder urothelial and smooth muscle cells on small intestinal submukosa: potential applications for tissue engineering technology. J Urol 2000-, 164:928-34
6 Cheng EY, Kropp BP. Urologic tissue engineering with smallintestinal subinucosa: potential clinical applications. World J Urol 2000; 18:26-30
7 Strehl R, Schumacher K, de Vries U, Minuth WW. Proliferating cells versus differentiated cells in tissue engineering. Tissue Eng 2002; 8:37-42
8 Voytik-Harbin SL, Rajwa B, Robinson JP. 3D imaging of extracellular matrix and extracellular matrix-cell interactions. Methods Cell Biol 2001; 63:583-97
9 Sittinger M, Bujia J, Rotter N, Reitzel D, Minuth WW, Burmester GR. Tissue engineering and autologous transplant formation: practical approaches with resorbable biomaterials and new cell culture techniques. Biomaterials 1996; 17:237-42
10 Fujiyama C, Masaki Z, Sugihara H. Reconstruction of the urinary bladder mukosa in three-dimensional collagen gel culture: fibroblast-extracellular matrix interactions on the differentiation of transitional epithelial cells. J Urol 1995; 153:2060-7
11 Erdani Kreft M, Sterle M. The effect of lamina propria on the growth and differentiation of urothelial cells in vitro. Pflugers Arch 2000; 440(5 Suppl):R181-2
12 Scriven SD, Booth C, Thomas DF, Trejdosiewicz LK, Southgate J. Reconstitution of human urotheliurn from monolayer cultures. J Urol 1997-,158:1147-52
13 Staack A, Alexander I, Merguerian P, Terris MK. Organ and species specificity in the stimulation of transitional epithelial cell growth by fibroblasts. Eur Urol 2001; 39:471-7
14 de Boer WI. Vermeij M, Diez de Medina SG, et al. Functions of fibroblast and transforming growth factors in primary organoid-like cultures of normal human urothelium. Lab Invest 1996; 75:147-56
15 Ludwikowski B, Zhang YY, Frey P. The long-term culture of porcine urothelial cells and induction of urothelial stratification. BJU Int 1999; 84:507-14
16 Rebel JM, De Boer WI, Thijssen CD, Vermey M, Zwarthoff EC, Van der Kwast TH. An in vitro model of urothelial regeneration: effects of growth factors and extracellular matrix proteins. J Pathol. 1994; 173:283-91
17 Langkilde NC, Wolf H, Orntoft TF. Lectinohistochemistrv of human bladder cancer: loss of lectin binding structures in invasive carcinomas. APMIS 1989:97:367-73.
18 Lindberg K, Badylak SF. Porcine small intestinal submukosa (SIS): a bioscaffold supporting in vitro primary human epidermal cell differentiation and synthesis of basement membrane proteins. Burns 2001; 27:254-66
19 Hutton KAR, Trejdosiewicz LK, Thomas DFM, Southgate J: Urothelial tissue culture for bladder reconstruction: an experimental study. J Urol 1993; 150:721-25
20 Sugasi S. Leshros Y. Bisson I, Zhang YY. Pavel K. Frey P: in vitro engineering of human stratified urothelium: analysis of its morphology and function. J Urol 2000: 164-.951-57

## Patentansprüche

1. Gewebetransplantatkonstrukt zur Rekonstruktion einer menschlichen oder tierischen Harnblase, umfassend
(a) eine biologisch kompatible, kollagenhaltige Membran; und
(b) auf der Membran eine oder mehrere Schichten von organ-spezifischen Gewebezellen,
wobei die äußere Schicht der organ-spezifischen Gewebezellen eine Schicht terminal differenzierter organ-spezifischer Gewebezellen ist und wobei die organ-spezifischen Gewebezellen Urothelzellen sind.

2. Gewebetransplantatkonstrukt nach Anspruch 1, **dadurch gekennzeichnet, daß** die Gewebezellen humanen oder porzinen Ursprungs sind.

3. Gewebetransplantatkonstrukt nach einem der vorstehenden Ansprüche, **dadurch gekennzeichnet, daß** die Gewebezellen autologen Ursprungs sind.

4. Gewebetransplantatkonstrukt nach einem der vorstehenden Ansprüche, **dadurch gekennzeichnet, daß** die biologisch kompatible, kollagenhaltige Membran ein submukosales oder dermales Gewebe ist.

5. Gewebetransplantatkonstrukt nach einem der vorstehenden Ansprüche, **dadurch gekennzeichnet, daß** die biologisch kompatible, kollagenhaltige Membran eine intestinale Submukosa, eine azelluläre Harnblasen-Submukosa oder eine azelluläre dermale Matrix ist.

6. Gewebetransplantatkonstrukt nach einem der vorstehenden Ansprüche, erhalten durch
(a) Aufbringen von Gewebezellen auf die biologisch kompatible, kollagenhaltige Membran;
(b) Proliferation der aufgebrachten Gewebezellen mittels stromaler Induktion in einem Kulturmedium unter Ausbildung einer oder mehrerer Schichten von Urothelzellen auf der Membran; und
(c) terminale Differenzierung der äußeren Schicht der expandierten Gewebezellen unter weiterer stromaler Induktion durch Verringerung mitogener Faktoren in dem Kulturmedium.

7. Verfahren zur Herstellung eines Gewebetransplantatkonstruktes zur Rekonstruktion einer menschlichen oder tierischen Harnblase, umfassend die Schritte
(a) Aufbringen von organ-spezifischen Gewebezellen auf eine biologisch kompatible, kollagenhaltige Membran;
(b) Proliferation der aufgebrachten Gewebezellen mittels stromaler Induktion in einem Kulturmedium unter Ausbildung einer oder mehrerer Schichten von Gewebezellen auf der Membran; und
(c) terminale Differenzierung zumindest der äußeren Schicht der expandierten Gewebezellen unter weiterer stromaler Induktion durch Verringerung mitogener Faktoren in dem Kulturmedium;
wobei die organ-spezifischen Gewebezellen Urothelzellen sind.

8. Verfahren nach Anspruch 7, umfassend die Schritte
(a) Aufbringen von Urothelzellen auf eine biologisch kompatible, kollagenhaltige Membran;
(b) Proliferation der aufgebrachten Urothelzellen mittels stromaler Induktion in einem Kulturmedium unter Ausbildung einer oder mehrerer Schichten von Urothelzellen auf der Membran; und
(c) terminale Differenzierung zumindest der äußeren Schicht der expandierten Urothelzellen unter weiterer stromaler Induktion durch Verringerung mitogener Faktoren in dem Kulturmedium.

9. Verfahren nach Anspruch 7 oder Anspruch 8, **dadurch gekennzeichnet, daß** die in Schritt (a) auf die Membran aufgebrachten Gewebezellen zur Proliferation und terminalen Differenzierung in einem Kulturmedium kultiviert werden, das fötales Rinderserum (FKS) oder autologes Serum enthält.

10. Verfahren nach einem der Ansprüche 7 bis 9, **dadurch gekennzeichnet, daß** die in Schritt (a) auf die Membran aufgebrachten Gewebezellen zur Proliferation und terminalen Differenzierung in einem Fibroblasten-konditionierten Medium (FBCM), das fötales Rinderserum (FKS) oder autologes Serum enthält, kultiviert werden.

11. Verfahren nach Anspruch 7, **dadurch gekennzeichnet, daß** es die Schritte
(a) Aufbringen von autologen Urothelzellen auf eine biologisch kompatible, kollagenhaltige Membran;
(b) Proliferation der aufgebrachten Urothelzellen in einem Fibroblasten-konditionierten Medium (FBCM), das fötales Rinderserum (FKS) enthält, bis vier bis sieben Schichten an Urothelzellen auf der Membran ausgebildet sind; und
(c) terminale Differenzierung der expandierten Urothelzellen der äußeren Schicht durch Verringerung der Konzentration an FKS
umfaßt.

12. Verwendung des Verfahrens nach einem der Ansprüche 7 bis 11 zur Herstellung eines Gewebetransplantatkonstruktes zur Rekonstruktion einer menschlichen oder tierischen Harnblase.

13. Verwendung nach Anspruch 12 zur Herstellung eines Gewebetransplantatkonstruktes nach einem der Ansprüche 1 bis 6.

## Claims

1. A tissue transplant construct for the reconstruction of a human or animal urinary bladder, comprising:
a) a biologically compatible, collagen-containing membrane; and
b) one or more layers of organ-specific tissue cells on the membrane,
where the outer layer of the organ-specific tissue cells is a layer of terminally differentiated organ-specific tissue cells and wherein the organ-specific tissue cells are urothelial cells.

2. The tissue transplant construct according to claim 1, **characterized in that** the tissue cells are of human or porcine origin.

3. The tissue transplant construct according any one of the preceding claims **characterized in that** the tissue cells are of autologous origin.

4. The tissue transplant construct according any one of the preceding claims **characterized in that** the biologically compatible, collagen-containing membrane is a submucosal or dermal tissue.

5. The tissue transplant construct according any one of the preceding claims **characterized in that** the biologically compatible, collagen-containing membrane is an intestinal submucosa, an acellular bladder submucosa, or an acellular dermal matrix.

6. The tissue transplant construct according to any one of the preceding claims obtained by
a) applying tissue cells to the biologically compatible, collagen-containing membrane,
b) proliferating the applied tissue cells by means of stromal induction in a culture medium to form one or more layers of urothelial cells on the membrane; and
c) terminally differentiating the outer layer of the expanded tissue cells with further stromal induction by reducing mitogenic factors in the culture medium.

7. A process for the preparation of a tissue transplant construct for the reconstruction of a human or animal urinary bladder, comprising the steps of:
a) applying organ-specific tissue cells to a biologically compatible, collagencontaining membrane;
b) proliferating the applied tissue cells by means of stromal induction in a culture medium to form one or more layers of tissue cells on the membrane; and
c) terminally differentiating at least the outer layer of the expanded tissue cells with further stromal induction by reducing mitogenic factors in the culture medium,
where the organ-specific tissue cells are urothelial cells.

8. The process according to claim 7, comprising the steps of:
a) applying urothelial cells to a biologically compatible, collagen-containing membrane,
b) proliferating the applied urothelial cells by means of stromal induction in a culture medium to form one or more layers of urothelial cells on the membrane; and
c) terminally differentiating at least the outer layer of the expanded urothelial cells with further stromal induction by reducing mitogenic factors in the culture medium.

9. The process according to claim 7 or claim 8, **characterized in that** the tissue cells applied to the membrane in step a) for proliferation and terminal differentiation are cultured in a culture medium that contains bovine embryo serum (BES) or autologous serum.

10. The process according to any one of claims 7 to 9, **characterized in that** the tissue cells applied to the membrane in step a) for proliferation and terminal differentiation are cultured in a fibroblast-conditioned medium (FBCM) that contains bovine embryo serum (BES) or autologous serum.

11. The process according to claim 7, **characterized in that** it comprises the steps of
a) applying autologous urothelial cells to a biologically compatible, collagencontaining membrane,
b) proliferating the applied urothelial cells in a fibroblast-conditioned medium (FBCM) that contains bovine embryo serum (BES) until four to seven layers of urothelial cells have been formed on the membrane; and
c) terminally differentiating the expanded urothelial cells of the outer layer by reducing the concentration of BES.

12. Use of the method according to any one of claims 7 to 11 for the preparation of a tissue transplant construct for the reconstruction of a human or animal urinary bladder.

13. The use according to claim 12 for the preparation of a tissue transplant construct according to claims 1 to 6.

## Revendications

1. Construction de transplantation tissulaire destinée à la reconstruction d'une vessie humaine ou animale, comprenant
(a) une membrane biologiquement compatible contenant du collagène; et
(b) une ou plusieurs couches de cellules tissulaires spécifiques à l'organe sur la membrane,
la couche extérieure des cellules tissulaires spécifiques à l'organe étant une couche de croissance de cellules tissulaires spécifiques à l'organe en phase de différenciation terminale et les cellules tissulaires spécifiques à l'organe étant des cellules urothéliales.

2. Construction de transplantation tissulaire selon la revendication 1 **caractérisée en ce que** les cellules tissulaires sont d'origine humaine ou porcine.

3. Construction de transplantation tissulaire selon l'une des revendications précédentes **caractérisée en ce que** les cellules tissulaires sont d'origine autologène.

4. Construction de transplantation tissulaire selon l'une des revendications précédentes **caractérisée en ce que** la membrane biologiquement compatible contenant du collagène est un tissu sous-muqueux ou cutané.

5. Construction de transplantation tissulaire selon l'une des revendications précédentes **caractérisée en ce que** la membrane biologiquement compatible contenant du collagène est une sous-muqueuse intestinale, une sous-muqueuse intestinale de vessie ou une matrice dermique acellulaire.

6. Construction de transplantation tissulaire selon l'une des revendications précédentes obtenue par
(a) application de cellules tissulaires sur la membrane biologiquement compatible contenant du collagène;
(b) prolifération des cellules tissulaires appliquées par induction stromale dans un milieu de culture sous formation d'une ou de plusieurs couches de cellules urothéliales sur la membrane; et
(c) phase de différenciation terminale de la couche extérieure des cellules tissulaires étendues sous une autre induction stromale par diminution de facteurs mitogènes dans le milieu de culture.

7. Processus de réalisation d'une construction de transplantation tissulaire destinée à la reconstruction d'une vessie humaine ou animale, comprenant les étapes:
(a) application de cellules tissulaires spécifiques à l'organe sur une membrane biologiquement compatible contenant du collagène;
(b) prolifération des cellules tissulaires appliquées par induction stromale dans un milieu de culture sous formation d'une ou de plusieurs couches de cellules tissulaires sur la membrane; et
(c) phase de différenciation terminale au moins de la couche extérieure des cellules tissulaires étendues sous une autre induction stromale par diminution de facteurs mitogènes dans le milieu de culture;
les cellules tissulaires spécifiques à l'organe étant des cellules urothéliales.

8. Processus selon la revendication 7 comprenant les étapes
(a) application de cellules urothéliales sur une membrane biologiquement compatible contenant du collagène;
(b) prolifération des cellules urothéliales appliquées par induction stromale dans un milieu de culture sous formation d'une ou de plusieurs couches de cellules urothéliales sur la membrane; et
(c) phase de différenciation terminale au moins de la couche extérieure des cellules urothéliales étendues sous une autre induction stromale par diminution de facteurs mitogènes dans le milieu de culture.

9. Processus selon la revendication 7 ou la revendication 8 **caractérisé en ce que** les cellules tissulaires appliquées dans l'étape (a) sur la membrane pour prolifération et phase de différenciation terminale sont cultivées dans un milieu de culture qui contient du sérum foetal bovin (SFB) ou du sérum autogène.

10. Processus selon l'une des revendications 7 à 9 **caractérisé en ce que** les cellules tissulaires appliquées dans l'étape (a) sur la membrane pour prolifération et phase de différenciation terminale sont cultivées dans un milieu conditionné par charges fibreuses (FBCM) qui contient du sérum foetal bovin (SFB) ou du sérum autogène.

11. Processus selon la revendication 7 **caractérisé en ce qu'**il comprend les étapes
(a) application de cellules urothéliales sur une membrane biologiquement compatible contenant du collagène;
(b) prolifération des cellules urothéliales appliquées dans un milieu conditionné par charges fibreuses (FBCM) qui contient du sérum foetal bovin (SFB) jusqu'à ce que quatre à sept couches de cellules urothéliales soient formées sur la membrane ; et
(c) phase de différenciation terminale des cellules urothéliales étendues de la couche extérieure par diminution de la concentration de SFB.

12. Utilisation du processus selon l'une des revendications 7 à 11 de production d'une construction de transplantation tissulaire destinée à la reconstruction d'une vessie humaine ou animale.

13. Utilisation selon la revendication 12 de production d'une construction de transplantation tissulaire selon l'une des revendications 1 à 6.
